# EUROPEAN PATENT APPLICATION

(11) **EP 4 431 082 A1**
(43) Date of publication of application: **18.09.2024**
(21) Application number: 23161630.1
(22) Date of filing: 13.03.2023
(51) Int. Cl.: A61K 9/00, A61K 9/08, A61K 47/10, A61K 47/18, A61K 47/12, A61K 35/06, A61K 31/00

(54) **AQUEOUS COMPOSITION COMPRISING BUDESONIDE**

(71) Applicant: Marinomed Biotech AG, 2100 Korneuburg (AT)
(72) Inventor: Prieschl-Grassauer, Eva, 1210 Wen (AT); Roch-Nakowitsch, Sabine, 3443 Sieghartskirchen (AT); Gössl, Andreas, 1190 Wien (AT); Sladek, Svenja, 1100 Wien (AT)
(74) Representative: Bogensberger, Burkhard

(57) **Abstract**

Disclosed is a buffered aqueous composition comprising solubilized budesonide, which has improved long-term storage stability and comprises alpha-escin as a saponin component, a buffer adjusted to an acidic pH of from 4 to 5, budesonide at a concentration of at least 100 *µ*g/mL, wherein the composition is free of dexpanthenol, and wherein the composition after storage at 20 - 25°C over a period of 12 months contains at least 90%, preferably at least 95 %, of the nominal starting concentration of solubilized budesonide, and no more than 1%, relative to the budesonide concentration, of total impurities including impurities R and D.

The invention further relates to the composition for use as a medicament in the treatment of inflammatory diseases or conditions.

## Description

### FIELD OF INVENTION

The present invention relates to an aqueous composition comprising solubilized budesonide and having improved storage stability.

### INTRODUCTION

WO2017009480 discloses a method of substantially increasing the solubility of poorly water-soluble hydrophobic drugs in aqueous solutions. The method comprises dissolving the hydrophobic drug in a suitable organic solvent and mixing the organic solvent with an aqueous buffer solution comprising a saponin component selected from the group of escin, glycyrrhizin and Quillaja saponaria extract, along with dexpanthenol and optionally further additives. The solubilization process is based on the formation of saponin micelles in a buffered aqueous environment.

One such poorly water-soluble drug is budesonide (CAS No. 51333-22-3), a glucocorticoid known inter alia as an agonist of glucocorticoid receptors and for having antiinflammatory activity. It is therefore frequently used to treat inflammatory conditions of the lungs and intestines such as asthma, COPD, Crohn's disease, or ulcerative colitis. The solubility of this drug in water being less than 30 µg/ml. Attempts to solubilize budesonide following the method disclosed in WO2017009480, i.e. dissolving budesonide in a phosphate buffered aqueous solvent comprising beta-escin, glycyrrhizin, polypropylene glycol, and dexpanthenol as essential ingredients, resulted in aqueous preparations comprising up to approximately 850 µg/ml. While this tremendous increase in solubilized drug was indeed stunning the long-term stability of the preparations was not fully satisfactory and resulted in the formation of undesired degradation and/or reaction products of part of the ingredients.

Therefore, there was a need to overcome this deficiency and to provide a stable aqueous preparation comprising a reasonably high concentration of dissoved or, more precisely, solubilized budesonide essentially without undesired impurities from decomposition and/or reaction products of some of the ingredients.

### DESCRIPTION OF THE INVENTION

Solubilized budesonide formulations prepared in accordance with the teaching of WO2017009480A1 contain dexpanthenol typically in a concentration range of from 0.5 to 5% (w/v). Dexpanthenol is used in these formulations to maximize the solubilization capacity and to stabilize the solubilization of budesonide against precipitation during storage at room temperature, i.e. 20 - 25 °C. As indicated above, following the teaching of WO2017009480A1 it is possible to solubilize up to about 850 µg/ml of budesonide and keep it solubilized for long-term storage.

However, evaluating the stability of long-term storage of a hydrophic drug in an aqueous solvent system does not only cover the determination of unimpaired maintenance of drug concentration througout the storage period but shall also include checking for the chemical stability of the entire solubilization system over the envisaged storage period.

Chemical degradation or decomposition of one or more of the ingredients of an aqueous buffered pharmaceutical composition, including the active pharmaceutical ingredient (API), as well as possible reaction products emerging as the result of chemical interactions between two or more of the ingredients and/or decomposition compounds can be stability limiting. Such degradation, decomposition, and/or reaction products are generally considered undesired impurities and present an impediment to obtaining marketing authorization, potentially limiting the availablity of useful therapies.

It is therefore imperative to remove such impurities to the extent possible from aqueous formulations intended for use as pharmaceutical compositions and/or to find ways to avoid the development of such impurities in aqueous solutions comprising solubilized drugs.

In connection with the solubilized budesonide formulations prepared in accordance with the teaching of WO2017009480A1 and including dexpanthenol as part of the solubilization system it was found that the long-term storage stability of the formulations was substantially impaired by the gradual development of an initially unknown and unexpected contaminant, hereinafter designated impurity R", and in addition, by a second contaminant, hereinafter referred to as "impurity D".

"Impurity D" is known in the art as 21-dehydro-budesonide, an oxidation product gradually emerging during storage of an aqueous budesonide formulation at ambient temperature. It is not generated by chemical interaction with dexpanthenol or other constituents of the formulation.

Contrary to impurity D, structural analysis of impurity R revealed that it is a reaction product of budesonide with 3-amino-1-propanol (3AP). 3AP is a known contaminant of commercially available dexpanthenol and may be present in concentrations of up to 0.5 % in samples conforming to the European Pharmacopoeia (EP), and up to 1.0 % in samples conforming to the United States Pharmacopoeia (USP). Furthermore, during storage of aqueous formulations containing dexpanthenol 3AP forms in a time- and temperature-dependent manner due to hydrolysis, following a reaction scheme indicated by Formula I. This hydrolysis reaction is relatively slow at neutral pH but accelerates at acidic or basic conditions.

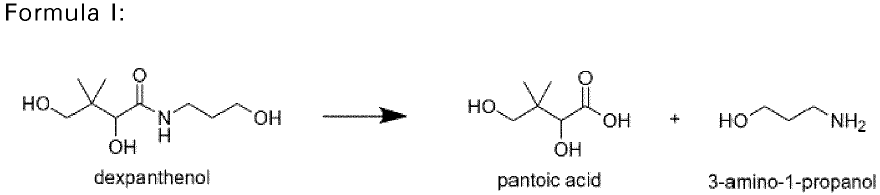

Where dexpanthenol is present in an aqueous solvent system together with budesonide the following chemical reaction takes place during prolonged storage (Formula II) resulting in the formation of 21-(3-hydroxypropyl)amino budesonide, i.e. impurity R. 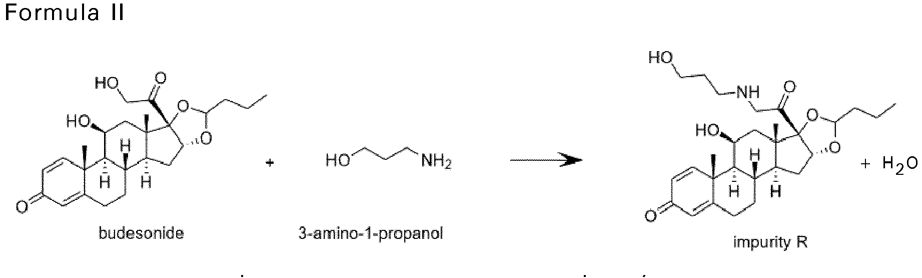 gated whether or not a reduction of the dexpanthenol concentration in the buffered aqueous composition or even a complete removal of dexpanthenol from the aqueous composition would cause substantial losses in drug concentration during storage. From WO2017009480A1 it was known that dexpanthenol has both a solubilizing as well as stabilizing effect on budesonide in a buffered aqueous solvent system.

Quite surprisingly, however, and contrary to expectations the reduction and even full elimination of dexpanthenol from the present experimental aqueous compositions did not induce precipitation of budesonide during storage at room temperature over several months. This surprising behaviour may be attributed to experimental variations of the present aqueous solubilizing system relative to the one disclosed in WO2017009480A1, namely comprising the preferred selection of alpha-escin as opposed to beta-escin as the saponin component, an experimental budesonide concentration of less than 850 µg/ml used in WO2017009480A1, preferably 100 - 400 µg/ml, or typically 200 µg/ml, and adjusting the aqueous formulation to a pH lower than pH 5.65 used in WO2017009480A1, preferably to a pH of from 4 to 5, and typically to a pH of 4.3.

Escin: While the current nomenclature of escins is based on a differentiation of the individual molecules contained in the mixture of components, the previously used nomenclature distinguished the mixtures of escin components based on their water solubilities. The main fraction of a naturally occurring escin mixture obtainable from horse chestnut extract consists of a compound having a protoescigenin backbone esterified with acetic acid at the C-22 position and with angelic/tiglic acid at the C-21 position (Geisler et al., 2019). This main fraction is called beta-escin. Besides beta-escin, two other fractions are identified in the escin mixture, namely alpha- and crypto-escin, wherein alpha-escin is a mixture of crypto- and beta-escin.

Beta- and crypto-escin differ in the position of the acetyl group in the backbone. Whereas in beta-escin this group is located at C-22, it can be found at C-28 in crypto-escin. Both forms can be distinguished by their solubilities in water, their melting points and their hemolytic indices. For example, crypto-escin is soluble in water whereas beta-escin is not readily soluble in water or buffered solutions having a pH of less than pH 5 (Geisler et al., 2019). On the other hand, alpha-escin being a 4:6 mixture of beta-escin and crypto-escin is better soluble in water than pure beta-escin. In addition, whereas alpha-escin remains stable in water or aqueous buffers in the absence of dexpanthenol, beta-escin precipitates in water or aqueous buffers after some hours in the absence of dexpanthenol, but not in the presence of dexpanthenol. It was therefore decided to elect alpha-escin as the preferred saponin component for solubilizing budesonide in the aqueous experimental samples.

### BRIEF DESCRIPTION OF THE FIGURES

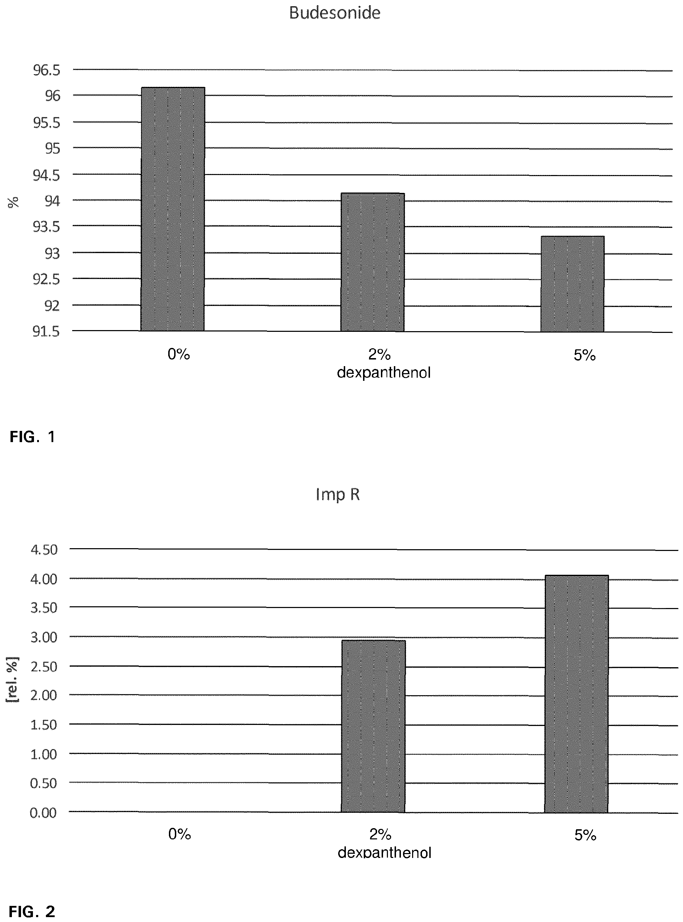

Figure 1: Representation of relative budesonide concentrations in aqueous buffered formulations containing solubilized budesonide, alpha-escin, and 0%, 2% or 5 % v/v dexpanthenol, expressed as percentage of recovered budesonide in the samples after 12 months of storage at 25°C, relative to the nominal starting concentration (i.e. 100%) of budesonide; analytical determination by HPLC; y-axis: concentration of recovered budesonide in % of the nominal starting concentration; x-axis: 0%, 2%, 5% v/v dexpanthenol.
Figure 2: Representation of contents of impurity R in aqueous buffered formulations containing solubilized budesonide, alpha-escin, and 0, 2 or 5 % dexpanthenol, determined by HPLC after 12 months of storage at 25°C; y-axis: relative concentration of impurity R expressed as % of corresponding recovered budesonide concentrations; x-axis: 0, 2, 5 % v/v dexpanthenol.

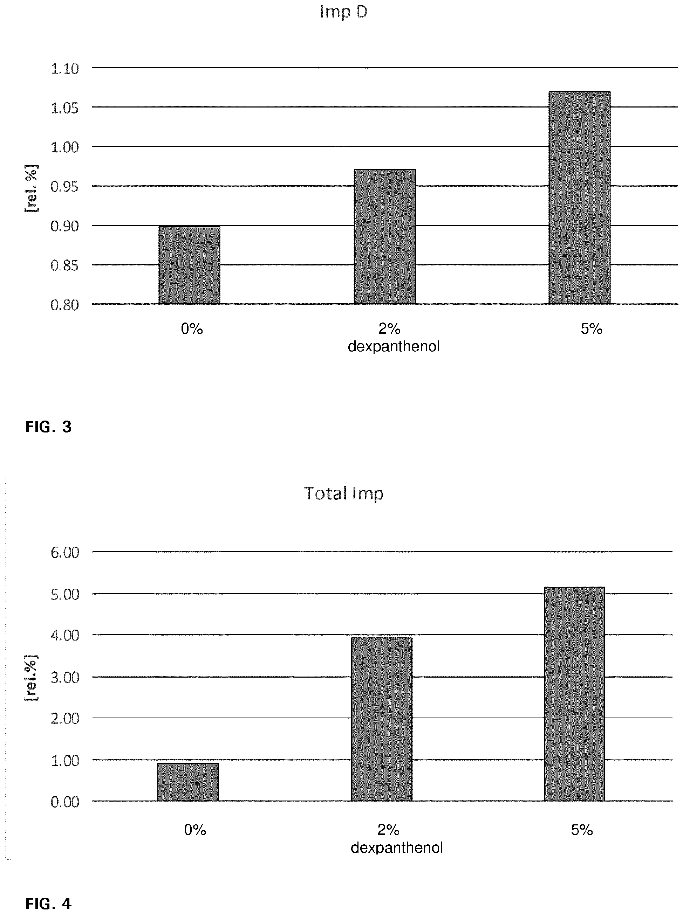

Figure 3: Representation of contents of impurity D in aqueous buffered formulations containing solubilized budesonide, alpha-escin, and 0, 2 or 5 % v/v dexpanthenol, determined by HPLC after 12 months of storage at 25°C; y-axis: relative concentration of impurity D expressed as % of corresponding recovered budesonide concentrations; x-axis: 0, 2, 5% v/v dexpanthenol.
Figure 4: Representation of contents of total impurities (including impurities "D" and "R") exceeding 0.1% in aqueous buffered formulations containing solubilized budesonide, alpha-escin, and 0, 2 or 5 % dexpanthenol, determined by HPLC after 12 months of storage at 25°C; y-axis: relative concentration of accumulated impurities including impurities R, D, and possible further impurities, expressed as % of corresponding recovered budesonide concentrations; x-axis: 0, 2, 5 v/v dexpanthenol.

### EXAMPLE 1: In situ formation of decomposition and/or reaction products during prolonged storage of aqueous solubilized budesonide compositions

**Table 1: Experimental composition**

| **Ingredient** | **Concentration [mg/ml]** |
|---|---|
| Budesonide | 0.2 |
| Propylene glycol | 104 (10% v/v) |
| Dexpanthenol | 0 - 50 |
| EDTA | 1.0 |
| alpha-Escin | 0.3 |
| Citric acid monohydrate | 10.4 |
| Trisodium citrate dihydrate | 14.85 |
| Water for injection | q.s. |
| HCl for pH adjustment | q.s. |
| pH | 4.3 |

All experimental formulations were stored in capped HDPE vials at 20-25°C.

As can be taken from Fig. 1, formulations comprising 200 µg/mL budesonide dissolved in a buffered aqueous solution comprising the ingredients listed in Table 1 and adjusted to a pH of 4.3, exert a concentration-dependent negative effect on the storage stability of the experimental samples. More specifically, while formulations without dexpanthenol stably maintained budesonide in solution over an observation period of 12 months at 20 - 25 °C, the addition of 2% v/v dexpanthenol caused a decline in recoverable budesonide from about 96% (no dexpanthenol) to about 94% (2% dexpanthenol), and the addition of 5% dexpanthenol caused a further decrease of recoverable solubilized drug down to about 93 % of the nominal starting concentration of budesonide.

At the same time, the concentration of "impurity R" increases with increasing dexpanthenol concentration, as depicted in Fig.2. Or in other words, eliminating dexpanthenol from the experimental samples resulted in the prevention of in situ formation of impurity R during storage at ambient temperature.

Furthermore, the reduction or elimination of dexpanthenol in these formulations not only reduced or eliminated the formation of impurity R in the experimental samples but also reduced the formation of impurity D (see Fig. 3). It was quite surprising that a concentration-dependent reduction of the formation of impurity D was observed with the experimental samples.

Without being bound by theory, this unexpected finding may possibly be due to the formation of impurity R in the presence of dexpanthenol, which impurity R, i.e. 3-amino-1-propanol (3AP), is a basic compound bearing a primary amine functional group. With increasing concentrations of 3AP in the experimental samples the pH also slightly increased despite the buffer system used in the solvent. Taking into account that the formation of the budesonide oxidation product (i.e. impurity D) is favored at higher pH, this might explain the observed effect of parallel increase or decrease of impurities R and D.

From this example it can further be concluded that providing an aqueous solvent system for solubilizing budesonide under the conditions defined in Table 1 allows for completely surrendering dexpanthenol as an obligatory ingredient for stably maintaining budesonide in a solubilized state over at least 12 monhts of storage at room temperature. As a beneficial side-effect of eliminating dexpanthenol from the aqueous budesonide formulations the in situ formation of undesired impurities R and D during storage of the liquid formulations is substantially reduced, as can also be taken from Fig. 4, which Fig. 4 is a graphic representation of the sum of all impurities determined by HPLC in the samples including impurities R and D.

## Claims

1. A buffered aqueous composition comprising budesonide solubilized therein, **characterized in that** it comprises
- a buffer adjusted to a pH of from 4 to 5, preferably adjusted to pH 4.3;
- propylene glycol at a concentration of from 5 to 15 %v/v, preferably of 10% v/v;
- escin, preferably alpha-escin, as a sole saponin component, at a concentration of from 0.1 - 1 mg/ml, preferably 0.3 mg/ml;
- EDTA at a concentration of from 0.5 - 2 mg/ml, preferably 1.0 mg/ml; and
- solubilized budesonide at a nominal starting concentration of at least 100 µg/mL, preferably at least 200 µg/mL, typically from 200 to 400 µg/mL;
wherein the composition is free of dexpanthenol, and wherein the composition after storage at 20 - 25°C over a period of 12 months contains at least 90%, preferably at least 95 %, of the nominal starting concentration of solubilized budesonide, and no more than 1%, relative to the solubilized budesonide concentration, of total impurities including impurities R and D.

2. The buffered aqueous composition of claim 1, **characterized in that** the buffer is a citrate buffer, a phosphate buffer, or a combination of both.

3. The buffered aqueous composition of claim 1 or 2, adapted for topical or systemic application, preferably adapted as a nasal spray, a mouth spray, a gargle solution, a liquid solution for inhalation, drops, a lotion, a cream, an ointment, or a foam.

4. The buffered aqueous composition of any one of claims 1 to 3, for use as a medicament, preferably for use as a medicament in the treatment of inflammatory diseases or conditions.

5. The buffered aqueous composition for use according to claim 4, wherein the inflammatory diseases or conditions are selected from inflammations of the respiratory pathways, of the lungs, of the intestines, of mucosal tissues, and of the skin.
